# EUROPEAN PATENT APPLICATION

(11) **EP 2 060 584 A1**
(43) Date of publication of application: **20.05.2009**
(21) Application number: 07020231.2
(22) Date of filing: 16.10.2007
(51) Int. Cl.: C07K 14/485, C07K 1/107, C12P 1/04

(54) **Method for the production of proteins with chemically functionalized N-termini**

(71) Applicant: Max-Planck-Gesellschaft zur Förderung der Wissenschaften e.V., 80539 München (DE)
(72) Inventor: Budisa, Nediljko, 85622 Feldkirchen (DE); Merkel, Lars, 81541 München (DE); Cheburkin, Yuri, 80798 München (DE); Ullrich, Axel, 80331 München (DE); Knyazev, Piotr, 82131 Stockdorf (DE); Wiltschi, Birgit, 85521 Ottobrunn (DE)
(74) Representative: Weiss, Wolfgang

(57) **Abstract**

The present invention refers to the production of N-terminally modified polypeptides, wherein an N-terminal methionine residue is replaced by a non-genetically encoded amino acid residue. The non-genetically coded amino acid residue may comprise a functional chemical moiety for coupling two heterologous molecules, e.g. reporter or effector molecules.

## Description

The present invention refers to the production of N-terminally modified polypeptides, wherein an N-terminal methionine residue is replaced by a non-genetically encoded amino acid residue. The non-genetically encoded amino acid residue may comprise a functional chemical moiety for coupling to heterologous molecules, e.g. reporter or effector molecules.

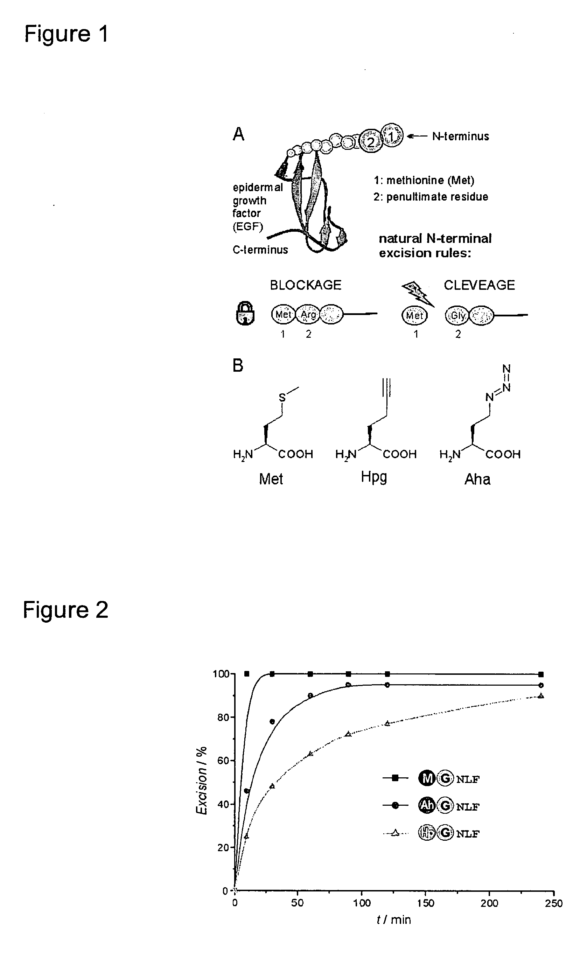

Protein synthesis initiates with methionine (Met) in the cytosol of eukaryotes and formylmethionine (fMet) in prokaryotes and eukaryotic organelles. N-terminal methionine excision (NME) is the major source of N-terminal amino acid diversity in all three life kingdoms ⁽¹⁾. The excision is dictated by the nature and bulkiness of the side chain of the second amino acid (penultimate residue) and is catalyzed by methionylaminopeptidases (MetAPs; EC 3.4.11.18) ^{(2,3)}. In bacteria, Lys, Arg, Leu, Phe and Ile protect N-terminal Met from removal, whereas Met excision is promoted by Gly, Ala, Pro, Cys, Ser, Thr or Val as the penultimate residues (Fig. 1A) ⁽⁴⁾. NME is an irreversible co-translational proteolysis, completed before the nascent polypeptide chains are fully synthesized. It was recently estimated that between 55 and 70% of the proteins of any given proteome undergo this Met-excision ⁽⁵⁾.

The canonical amino acid Met is recognized, activated and charged onto its cognate tRNAs by methionyl-tRNA synthetase (MetRS). The N-formyl group from initiator methionine is enzymatically removed from the nascent polypeptide in bacteria. This is an essential prerequisite for the posttranslational action of MetAP ⁽⁶⁾. *E. coli* MetRS exhibits a remarkable flexibility in substrate binding and tRNA charging - a fact that we and others have used for co-translational incorporation of a large number of Met analogs and Met-like amino acids (surrogates) into polypeptide sequences ^{(7,8)}. The chemical diversification of Met side chains achieved in this way is quite impressive, ranging from aliphatic, chalcogen- and halogen-containing side chains to unsaturated chemical groups like alkenes or alkynes and other interesting biobioorthogonal groups such as azides ^{(9,10)}.

It is generally known that only the specialized transfer RNA (tRNA) for translation initiation - tRNA^{fMet} in eubacteria and TRNN^{iMet} in other organisms - can be used to start translation. Both initiator and elongator tRNA^{Met} are aminoacylated by the same MetRS.

Currently available methods for modifications of translation initiations are based on in vitro expression systems. First method include the introduction of fluorophores (fluorescein, BODIPY), of affinity tags such as biotin as well as other labels at the N-terminus of proteins is based on the use of an *Escherichia coli* initiator tRNA^{fMet} misaminoacylated with methionine modified at the α-amino group (25). A second method for in vitro N-terminal labeling uses chemically aminoacylated amber initiator suppressor tRNA and a DNA template which contains a complementary amber (UAG) codon instead of the normal initiation (AUG) codon (26). Finally, most sophisticated in vitro method include use of sequences whose leader sequence cleveage yiled Lys at N-terminus. Enzymatically preacylated tRNAs were mixed with enzyme from intermediary metabolism (Leu/Phe-tRNA transferase) in order to get suitable labelling at target protein N-terminus (27).

Previously, Tirrell and co-workers demonstrated near-quantitative substitution of the Met residues in dihydrofolate reductase by homopropargylglycine (Hpg) ^{(11,12)}. The initiator Met was also substituted but Hpg in the first position was not excised. Similarly, we globally replaced Met with trifluoromethionine in green fluorescent protein and discovered an efficient blockage of the N-terminal excision of trifluoro-Met with Ser as the penultimate residue ⁽¹³⁾.

An object of the present invention was to modify the NME rules in recombinant polypeptides by incorporating different Met analogues at the protein N-terminus.

According to the present invention a method is provided for producing gene-encoded modified polypeptides by replacing an N-terminal or initiator methionine residue with a different, particularly with a non-genetically encoded amino acid. The incorporation of amino acids different from methionine into the N-terminus of the polypeptide may modify, e.g. increase or decrease N-terminal amino acid excision during translation and preferably allows subsequent introduction of heterologous molecules, e.g. by biobioorthogonal transformation reactions.

A first aspect of the present invention refers to a method of producing an N-terminally modified polypeptide by translation from an RNA molecule in a host cell or in an in vitro translation system, wherein the RNA molecule encodes a polypeptide comprising an N-terminal methionine residue, wherein the translation occurs at conditions under which the N-terminal methionine residue is replaced by a non-genetically encoded amino acid residue and wherein thereby an N-terminal amino acid excision of the polypeptide is at least partially modified.

A further aspect of the present invention refers to a method of producing an N-terminally modified polypeptide by translation from an RNA molecule in a host cell or in an in vitro translation system, wherein the RNA molecule encodes an N-terminal polypeptide portion comprising an amino acid sequence which allows proteolytic cleavage and wherein the first amino acid residue behind the cleavage site is the amino acid residue to be modified, wherein the translation occurs at the conditions under which the amino acid residue to be modified is replaced by a non-genetically encoded amino acid residue, and wherein the polypeptide is subjected to a proteolytic cleavage at the cleavage site.

Still a further aspect of the present invention refers to a method of modifying efficacy of translation initiation, wherein an RNA molecule encoding a polypeptide comprising an N-terminal amino acid residue is translated in a host cell or in an in vitro translation system, wherein the translation occurs at conditions under which the N-terminal methionine residue is replaced by a non-genetically encoded amino acid residue and wherein thereby the efficacy of translation initiation is modified, e.g. decreased or increased.

Still a further aspect of the present invention refers to an N-terminally modified polypeptide comprising at its N-terminus a non-genetically encoded amino acid residue. This polypeptide may be obtained by translation from an RNA molecule in vivo or in vitro, particularly by the method of the present invention.

The present invention refers to the production of modified polypeptides, wherein a methionine residue, particularly an N-terminal methionine residue (i.e. an initiator methionine residue) is replaced by a non-genetically encoded amino acid residue. The polypeptide is produced by translation from an RNA-molecue, particularly from an mRNA molecule in a host cell or in an in vitro translation system. The mRNA may be obtained by transcription from a chromosomal gene or from an extrachromosomal gene, e.g. gene located on a plasmid vector. The host cell may be a prokaryotic or eukaryotic cell, particularly a bacterial cell, more particularly a gram-negative bacterial cell, such as E.coli. Further preferred host cells are eukaryotic cells, e.g. fungus cells, such as yeast, or animal cells, such as mammalian cells. Alternatively, the translation may be carried out in an in vitro translation system , e.g. a non-cellular system comprising the components required for translation, e.g. a eukaryotic or prokaryotic in vitro translation system. Examples of suitable in vitro translation systems are described in the Book "Cell Free Protein Expression" edited by James R. Swartz (Springer Verlag, Heidelberg, 2003). In addition to this classical expression platform, other, more sophisticated in vitro-based systems, might be used for initiator residue change such as Pure Translation System (PURE) (28) and PURE-based ribosome display (29, 30), translation display (31) and ribosomal synthesis of unnatural peptides (i.e. Peptidomimetica in the genetic format) (32).

An efficient replacement of the N-terminal methionine residues by a non-genetically encoded amino acid residue may be effected by the following means:

The non-genetically encoded amino acid residue may be provided in the culture medium of the host cell and taken up by the host cell via active transmembrane transport, for example, cells grown in rich or minimal media may accumulate non-genetically encoded amino-acids in the cytosol at least 10-fold when compared with the concentration of the amino-acid in the culture medium. The concentration of the non-genetically encoded amino acid in the cytosol is sufficient for efficient incorporation into polypeptides during translation.

An alternative way to deliver non-genetically encoded amino acid into host cell cytosol at the amount sufficient enough to enable its efficient translation is to use methods of metabolic engineering. It is well known that living beings have remarkable capacity to produce a wide variety of secondary metabolites, among them more than 700 known biogenic amino acids (33, 34). These represent a large natural reservoir of potential substrates for protein biosynthesis with an expanded genetic code. The genes of synthetic pathways of these amino acids can be introduced into desired host cells and coupled with their incorporation at the initiator position of the target polypeptide sequence.

Alternatively or additionally, the host cell may be a methionine-auxotrophic host cell, wherein the metabolic pathway supplying the cell with methionine, is switched off. The auxotrophic cell is preferably grown in a minimal culture medium containing limited concentrations of methionine, while transcription of the target gene is repressed. At a predetermined culture stage, e.g. at the onset of the stationary growth phase, the reservoir of methionine is depleted. At said point of time, the non-genetically encoded amino acid is added to the culture medium and the expression of the target gene is induced. This results in an accumulation of a polypeptide, wherein the N-terminal methionine residue is replaced by the non-genetically encoded amino acid residue.

Alternatively or additionally an externally added substance, e.g. a metabolic inhibitor of the methionine synthesis, can deactivate the synthesis of methionine, providing in this way a control of the methionine supply. Further, cellular components involved in the synthesis, turnover and transport of methionine may be genetically and/or physiologically modified, so that the methionine supply is efficiently controlled either in a whole cell or in cell compartments.

Alternatively, non auxotrophic host cells may be used without the need of methionine supply control. For example, introduction of heterologous translational components, particularly bioorthogonal amino acids: tRNA pairs, from phylogenetically unrelated organisms are specifically capapble to decode initial methionine codons (AUG, GUG, CUG) or even non-triplet coding units. For example, prokaryotic host cells may be supplied with natural or engineered archaeal or eukaryotic translational components, whereas archaeal or eukaryotic host cells may be supplied with imported eukaryotic translational initiation components that are completeable with the host cell expression machinery.

Alternatively, an in vitro synthesis may be carried out using cell extracts and/or reconstituted translational systems which allow a precise control of aminoc acid supply as well as control of target gene expression. These in vitro translations ystems may be supplied by tRNA species, e.g. chemically amino ethylated tRNA species capable of decoding iMet as well as other translational components.

In one embodiment, the polypeptide, which is produced according to the present invention may comprise a single methionine residue which is the N-terminal methionine residue. In a different embodiment, the polypeptide may comprise an N-terminal methionine residue and at least one internal methionine residue. In this case, only the N-terminal methionine residues may be replaced by non-genetically encoded amino acid or the N-terminal methionine residue and at least one of the internal methionine residues may be replaced by non-genetically encoded amino acid residues.

The replacement of the N-terminal methionine residue may result in a modification of the N-terminal amino acid excision of the polypeptide. In one embodiment, the N-terminal amino acid excision may be increased, wherein e.g. an increase of N-terminal amino acid excision of at least 20%, preferably at least 50%, and more preferably of at least 100% compared to the non-modified polypeptide is obtained. In a different embodiment, the N-terminal amino acid excision may be decreased, wherein e.g. a decrease of N-terminal amino acid excision of at least 20%, preferably of at least 50% and more preferably of at least 90%, compared to the non-modified polypeptide is observed.

According to the present inventon, it was also found that the penultimate amino acid residue, i.e. the amino acid residue immediately after the N-terminal amino acid residue may affect N-terminal amino acid excision and/or polypeptide stability, even when the N-terminal methionine amino acid residue is replaced by non-genetically encoded amino acid residue. In one embodiment of the present invention, the penultimate amino acid residue is an amino acid residue which promotes N-terminal amino acid excision, which is particularly selected from the group consisting of the amino acid Gly, Ala, Pro, Cys, Ser, Thr and Val. In a further embodiment, the penulatimate amino acid residue may be an amino acid residue which increases polypeptide stability, e.g. selected from the group consisting of Met, Gly, Ala, Ser, Thr and Val.

In a different embodiment, the penultimate amino acid residue may be amino acid residue which inhibits N-terminal amino acid excision, particularly selected from the group consisting of Arg, Lys, Leu, Ile and Phe.

Furthermore, the penultimate amino acid residue may be an amino acid residue which decreases polypeptide stability (e.g. measured by half-life time as described in references 35 and 36), which e.g. may be selected from the group consisting of Arg, Lys, Asp, Leu, Ile and Phe.

In addition to the penultimate residue, the removal of N-terminal amino acids may be also influenced by the antepenultimate residue, the third residue after Met. Extensive study using model peptides and mutant MetAPs was performed by Liao et al. (2004) (Ref.: Liao,Y-D., Jeng, J-C., Wang, C-F., Wang , S-C. and Chang , S.T. (2004). Removal of N-terminal methionine from recombinant proteins by engineered E. coli methionine aminopeptidase. Protein Science, 13, 1802-1810.).
Their major conclusions are:
- Mutations of the penultimate or antepenultimate residues of any desired target protein may also alter their stability and function.
- N-terminal Met with penultimate Ala, Gly, Ala, Pro, Cys, Ser, Thr or Val is mainly efficiently removable when the antepenultimate residue is small or moderate, such as Gly, Ala, Ser, Thr, Val, or Asn, but not bulky or charged, such as Asp, Glu, Phe, Trp, Lys, or Arg
- Pro residue at the antepenultimate site may exert hindrance on the N-terminal-Met removal.
- There are differences in activity on E. coli versus human MetAPs with respect of penultimate residue as well as antepenultimate residues.

Thus, the ante-penultimate amino acid residue, i.e. the amino acid residue which is the second after the N-terminal amino acid residue, may affect the N-terminal amino acid excision and/or polypeptide stability. For example, the ante-penultimate amino acid residue may be selected from medium-size hydrophobic residues such as Leu, Ile or Val, or from medium-size hydrophilic residues such as Glu, Asp or Thr.

In a preferred embodiment of the present invention, a non-genetically encoded amino acid residue, which replaces the N-terminal methionine residue, comprises a functional chemical moiety, preferably in its side chain, but backbone modifications are also possible, e.g. α-methyl methionine. The term "functional chemical moiety" in this context preferably refers to a moiety which is reactive with a complementary functional chemical moiety, particularly under physiological conditions, i.e. aqueous milieu, about neutral pH (e.g. pH between 4 and 8.5) and temperatures between 20° and 40°C, e.g. 37°C. Preferably, the functional chemical moiety is an biobioorthogonal moiety, i.e. a moiety that does not appear or only appears in exceptional cases (such as e.g. in the secondary metabolisms of some plants or fungi) in cellular systems. Preferred bioorthogonal moieties do not have substantial cross-reactivity with cellular components, particularly with naturally occurring amino acid side chains and other cellular substances.

Preferred examples of functional bioorthogonal moieties are such as azide groups, unsaturated groups, e.g. alkene or alkyne groups, phosphine groups, chalcogen groups, e.g. ethers, ketones, aldehydes, thioethers, thioketones or thioaldehydes, thioamines, various methyl esters or thioesters, or halogen moieties, particularly chloro, bromo or iodo moieties. These compounds may undergo specific bioorthogonal chemical reactions such as cycloaddition reactions, e.g. an [1,3]-dipolar cycloaddition between an azide group and an alkyne group catalyzed by copper ions ("click chemistry"), reactions between halogen moieties and unsaturated carbon-carbon bonds, such as the different variants of Heck-additions, e.g. Sonogashira or Mizoroki, in the presence of a suitable catalyst, such as palladium, Staudinger reactions, i.e. reactions between azine and phosphine groups and variations of naturally occurring chemical reactions, e.g. modifications of thiol/thiolester exchanges.

In a preferred embodiment, the present invention comprises in a further step the coupling of a heterologous molecule to the functional chemical moiety of a non-genetically encoded amino acid residue. The heterologous molecule may be for example a reporter molecule, e.g. a molecule comprising a detectable group, particularly an optically detectable group, such as a fluorescent group, a luminescent group or a dye. Alternatively, the heterologous molecule may be an effector molecule, i.e. a molecule which modifies physiological, biological and/or pharmacological properties of the polypeptide. For example, the effector group may be a polymer group, such as a polyethylene glycol group, e.g. a polyethylene glycol group with a molecular weight between 500 and 20.000. Furthermore, the effector molecule may be a carbohydrate group, e.g. a naturally or non-naturally occurring glycosyl residue. Furthermore, the effector molecule may be a pharmacologically active substance, such as a cytotoxic agent, an alkaloid, an antibiotic, etc.. Further examples of effector molecules are heterologous peptides or polypeptides, nucleic acids or lipids. In a still further embodiment, the modified polypeptide may be bound to a solid phase, e.g. a particle, bead, chip, medical device etc. This embodiment is particularly suitable for the manufacture of polypeptide arrays, wherein a plurality of different polypeptides is immobilized via their N-termini to different sites of a solid phase, e.g. a biochip. Still further examples are substances interesting for material or nano-sciences such as polymers, quantum dots, various metal complexes, fullerenes, redox, light, or osmotic sensors and so on.

The non-genetically encoded amino residue is preferably an N-methionine analogous amino acid such as homopropargyl glycine (Hpg), azidohomoalanine (Aha), norvaline, norleucine, ethionine, crotylglycine, 2-amino-hex-5-ynoic acid, 6,6,6-trifluoronorleucine, 2-amino-4,4,4-trifluorobutyric acid, C-propargylglycine, allylglycine, beta-cyclopropyl alanine, α-hydroxymethionine, or photoactive diazirine methionine analogues. Particularly preferred are the L-enantiomers of these amino acids. Especially preferred are Hpg and Aha. Alternatively, the non-genetically encoded amino acid residue may also be non-homologous to methionine, such as glutamate-gamma-methyl ester or aspartate-beta-methyl ester and particularly the L-enantiomers thereof.

The polypeptide which is modified according to the present invention may be any polypeptide which may be produced by translation in a host cell or in an in vitro translation system. Preferred examples of polypeptides are growth factors, such as fibroblast growth factors (FGFs), e.g. basic fibroblast growth factor (bFGF or FGF2), epidermal growth factor (EGF), erythropoietin (EPO), granulocyte-colony stimulating factor (G-CSF), granulocyte-macrophage colony stimulating factor (GM-CSF), growth differentiation factor-9 (GDF9), hepatocyte growth factor (HGF), insulin-like growth factors (IGFs), cytokines (Interferons; Interleukins), myostatin (GDF-8), nerve growth factor (NGF), neurotrophins, platelet-derived growth factor (PDGF), thrombopoietin (TPO), transforming growth factor beta (TGF-β) and tumor necrosis factor (TNF), viral proteins, such as hepatitis B virus surface antigens or HIV proteins, annexins, such as annexin V, structure polypeptides, such as collagens, antibodies, such as trastuzumab and related molecules, such as antibody fragments, e.g. Fab-fragments, intercalins, chromobodies or diabodies, and others, such as barstar, G-protein coupled receptors, green fluorescent protein and its spectral variants, human superoxide dismutase (hSOD1), spider silk and extracellular matrix proteins. In addition, proteases, kinases, phosphatases, hydroxylases, ligases or polymerases may be modified as well.

In a further embodiment, the polypeptide may comprise an N-terminal polypeptide portion comprising an aminio acid which allows proteolytic cleavage and wherein the first amino acid residue behind the cleavage site is the amino acid residue to be modified. In this embodiment, the amino acid residue to be modified is preferably a methionine residue. Further, it is preferred that the amino acid residue to be modified only occurs once in the polypeptide after cleavage of the N-terminal polypeptide portion. Alternatively, the ploypeptide may contain more than one amino acid residue to be modified after cleavage.

The present invention also provides novel N-terminally modified polypeptides comprising at their N-termini a non-genetically encoded amino acid residue, preferably a non-genetically encoded amino acid residue comprising a functional chemical moiety. The functional chemical moiety may be coupled to a heterologous molecule as described above.

Further, the present invention is explained by the following Figures and Examples hereinbelow.

Figure 1: The structure of mature human epidermal growth factor (hEGF) with marked N-, and C-terminus, along with the simple NME rules when Gly or Arg are the penultimate residues. B) Chemical structures of methionine (Met) and its analogs, azidohomoalanine (Aha) and homopropargylglycine (Hpg).

Figure 2: Kinetics of the *in vitro* excision of N-terminal Met, Aha and Hpg by MetAP. The pentapeptides Met1-Gly2-Gln3-Leu4-Phe5; Ahal-Gly2-Gln3-Leu4-Phe5; and Hpgl-Gly2-Gln3-Leu4-Phe5 were incubated with *E.coli* MetAP and the cleavage products at different time points analyzed by HPLC. Each time point was measured two times. Ah, Aha; Hp, Hpg, canonical amino acids in one letter code; data points are connected by trend lines.

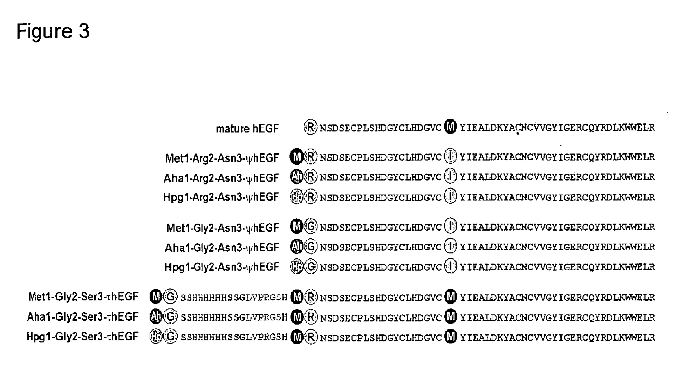

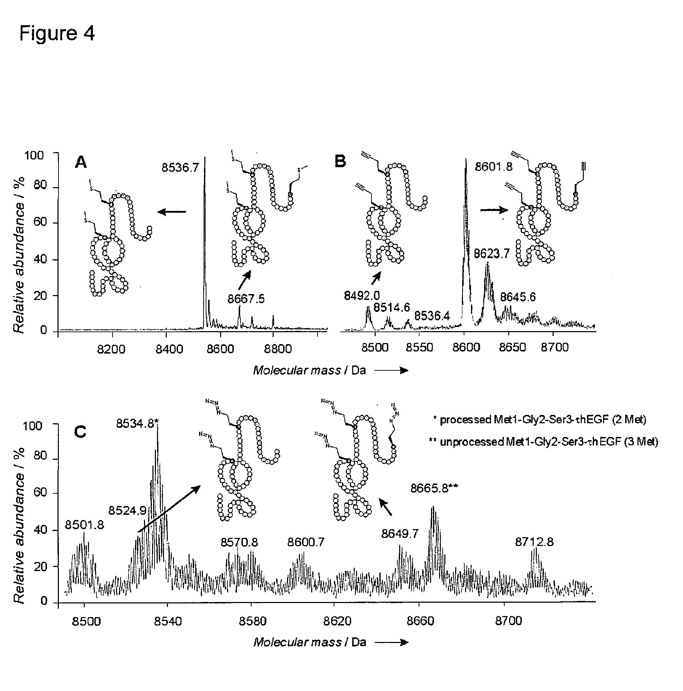

Figure 3. Met, Aha and Hpg variants of the hEGF mutants used in this study. Ah, Aha; Hp, Hpg; HHHHHH, hexahistidine tag; LVPRGS, thrombin Figure 4. Mass spectrometry of Met1-Gly2-Ser3-τhEGF (A), Ahal-Gly2-Ser3-ThEGF (B) and Hpg1-Gly2-Ser3-τhEGF (C). See text for discussion of the determined masses. The following mass peaks were assigned to protein species containing N-terminal 2,4-dyaminobutyric acid (DAB) in combination with Met: 8501.8 Da (processed τHEGF - 1 Met + 1 DAB); 8570.8 Da (unprocessed τhEGF + 3 DAB) and 8600.7 Da (unprocessed τhEGF + 1 Met + 2 DAB). The peak corresponding to the mass of 8712.8 Da was not identified. We generally observed that Aha-containing proteins purified via Ni-NTA resin columns tend to exhibit a higher azide → amine reduction than when purified by other methods.

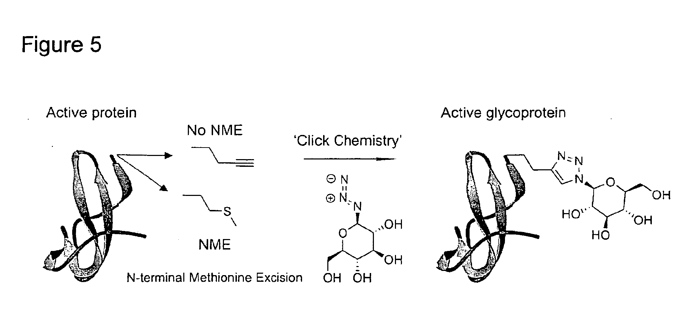

Figure 5. Scheme depicting an example for N-terminal gylcosylation of Hpg-containing human Epidermal Growth Factor (hEGF) generally demonstrates the possibility to couple NME rule modification with the subsequent posttranslational modification in a controlled manner. In particular, the substitution of Met-residues with Hpg in proteins with Nt-sequence Met(1)-Gly(2), efficiently blocks N-terminal processing. At the same time the alkyne of Hpg enables for its chemical derivatization by conjugation with different azide containing ligands via 'click chemistry'. In this way, human epidermal growth factor was successfully glycosylated without losing its biological activity. By chemically controlled posttranslational modifications of the protein N-terminus, the metabolic fate as well as the life-span of a target protein can be changed at will.

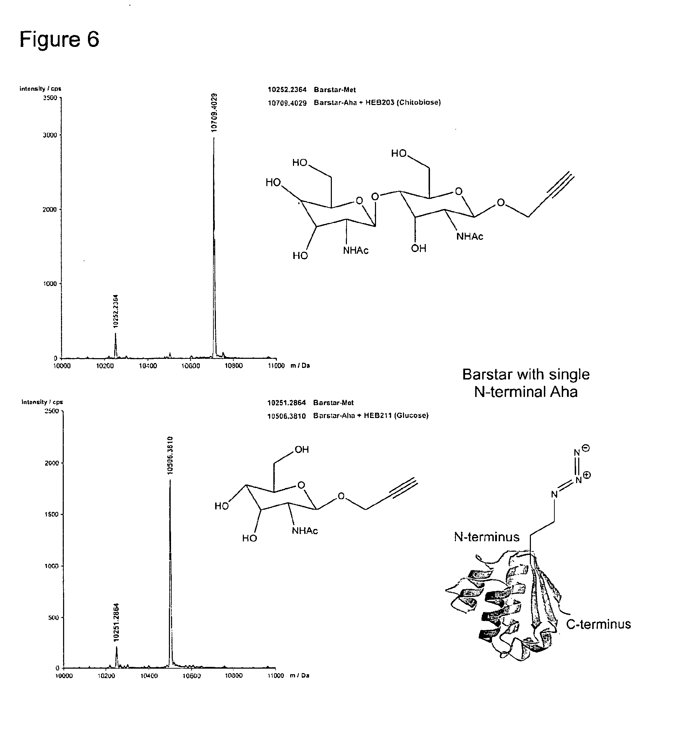

Figure 6. Chemical glycosylation of N-terminal Aha-containing barstar. The copper catalyzed cycloaddition reaction between Aha-barstar and the alkyne-sugars chitobiose (upper part) and NHAc-Glucose (lower part) yielded single molecular species whose glycosylation was detectable by electrospray-ionisation mass spectrometry (ESI-MS). In fact, rigorous ESI-MS analyses revealed quantitative glycosylations of the Aha-barstar N-terminus. Remaining minor peak (10251 Da) in both cases is non-derivatized native N-terminal Met-containing protein.

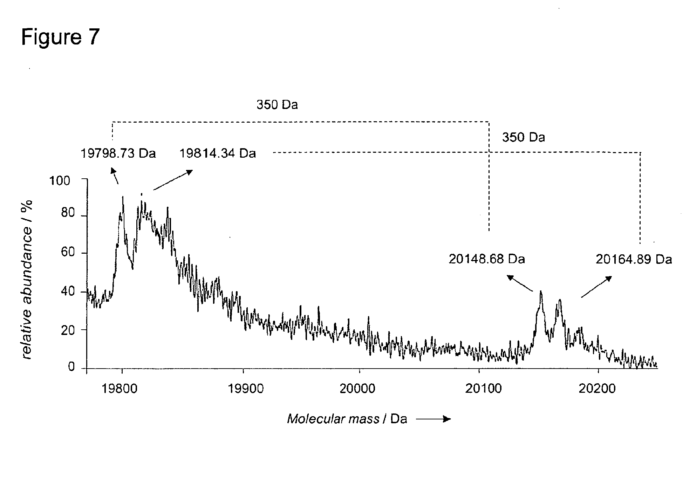

Figure 7. PEGylation of soluble Fc gamma receptor (sFc gammaRIII, CD16). Primary sequence sFc gammaRIII consists of 177 amino acids, including single N-terminal Methionine residue (Met1). The replacement Met1→Hpg1 enabled for Nt-terminal conjugation of Azido-PEG350 (alpha-Amino-omega-Azido-dodeca(ethylene glycol)); Mw=350.42 kDa) via click chemistry. The masses of native (i.e. Met-containing), derivatized (i.e. Hpg-containing; 19798.73 and 19814.34 Da, respectively) and conjugated (i.e. PEG-containing; 20148.68 and 20164.89 Da, respectively) proteins are heterogeneous most probably due to spontaneous deamidation (± 16 Da). Nonetheless, the mass shift of exactly 350 Da in the presented spectrum clearly indicates successful PEGylation of Fcgamma receptor IIb.

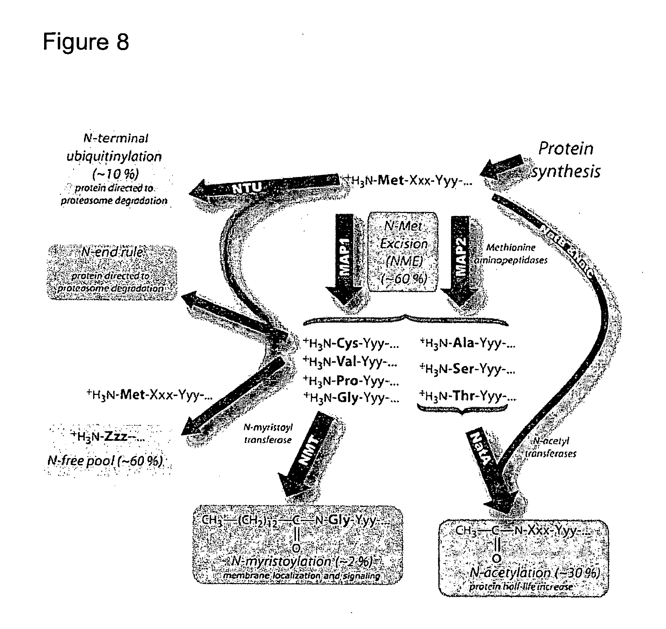

Figure 8: General importance of Nt protein processing in living cells. The removal of N-terminal Met and the subsequent exposure of small residues, such as Gly, Ala, Pro, Cys, Ser, Thr or Val, protects the processed proteins from degradation. As predicted from the N-end rule of protein stability, proteins possessing a bulky N-terminal residue, known as destabilizing residues (Lys, Arg, Leu, Phe, Ile), have short half-lives and those possessing small terminal residues, known as stabilizing residues, have long half-lives (VARSHAVSKY, 1996). From: The Proteomics of N-terminal Methionine Cleavage Mol. Cell. Proteomics 2006, 5, pp.2336 (F. Frottin *et al*.).

### Example 1: N-terminal modification of EGF

### 1. Material and Methods

All chemicals were from Sigma (Steinheim, Germany) or Merck KGaA (Darmstadt, Germany) unless otherwise indicated.

**1.1. Peptide synthesis.** Solvents in p.a. quality were from Fluka (Buchs, Switzerland), Merck KGaA or Riedel-de Haën (Seelze, Germany). DMF was from Carl Roth GmbH (Karlsruhe, Germany) and the resin for SPPS purchased from Rapp Polymere (Tübingen, Germany). L-Hpg for the generation of Fmoc protected Hpg was a generous gift of Chiralix (Nijmegen, The Netherlands). All other protected amino acids were from Iris Biotech (Marktredwitz, Germany).

L-Aha was synthesized according to ⁽²²⁾ with some minor alterations: (S)-(+)-2-Amino-4-bromobutyric acid served as the starting material. After protection of the acid function as the methyl ester, the azide group was introduced as described in ⁽²²⁾. Deprotection was achieved by 1.1 eq of NaOH and the product was purified by cation exchange chromatography (Dowex 50-WX8, 100 mesh). The yield of about 30 % L-Aha was slightly better than that described in the literature.

The synthesis of peptides H₂N-Gly-Asn-Leu-Phe-CONH₂, H₂N-Met-Gly-Asn-Leu-Phe-CONH₂, H₂N-Aha-Gly-Asn-Leu-Phe-CONH₂, H₂N-Hpg-Gly-Asn-Leu-Phe-CONH₂, NH₂-Arg-Asn-Leu-Phe-CONH₂, NH₂-Met-Arg-Asn-LeuPhe-CONH₂, NH₂-Aha-Arg-Asn-Leu-Phe-CONH₂ and NH₂-Hpg-Arg-Asn-LeuPhe-CONH₂ followed the Fmoc/tBu strategy. Peptides P1 and P2 were synthesized on a continuous-flow synthesizer Pinoneer Peptide Synthesis System Model GEN600611 (PerSeptive Biosystems Inc, Framingham, MA). Subsequent coupling of Fmoc-Met, Fmoc-Aha, Fmoc-Hpg, deprotection and cleavage from the resin were performed manually following standard protocols. All peptides were dissolved in water containing 28.6 % DMSO in order to obtain a final concentration of 1 % in the *in vitro* MetAP assay mix (see below).

***1.2. In vitro* MetAP assay.** The *in vitro* excision of N-terminal amino acids by methionylaminopeptidase was performed according to ⁽²³⁾. Briefly, in a total volume of 400 µl, the assay mix contained 0.2 mM CoCl₂, 0.1 M Na₂HPO₄/NaH₂PO₄ pH 7.5, 1.8 mM peptide or protein, 1 % DMSO and 0.43 µM *E.coli* MetAP. The mixture was incubated at 37 °C with vigorous agitation. After varying incubation times, the reaction was stopped by mixing 40 µl of the assay mix with 10 µl of 1 M HCl. All samples were analyzed by analytical HPLC and LC-MS.

**1.3. Peptide analysis.** Analytical HPLC of the peptides before and at different time points during MetAP treatment was performed on a Waters GmbH (Eschborn, Germany) HPLC system equipped with a controller 600, pumps 626 with inline-degasser AF, autosampler 717ₚₗᵤₛ, data module and dual A absorbance detector 2487. Peptides were separated on an ET 125/4 Nucleosil 100-5 C8 column (Macherey & Nagel, Düren, Germany) using a gradient from 95 % A (0.1 % TFA in water) to 90 % B (0.08 % TFA in acetonitrile) within 18 minutes at a flow rate of 1.5 ml/min. The absorbance was detected at 210 nm.

For LC-MS, 20 µl aliqouts from selected time points were pre-separated on an X-Bridge C8 column (5 µm, 100 x 2.1 mm, Waters GmbH) by eluting with a gradient from 90 % A (0.05 % TFA in water) to 90 % B (0.05 % TFA in acetonitrile) within 15 minutes at a flow rate of 250 µl/min. The LC system was equipped with a microgradient pump system 140 C (Applied Biosystems Perkin-Elmer Corp), an autosampler series 200 (Perkin Elmer, Üblingen, Germany) and a UV/Vis detector series 1100 (Agilent Technologies, Palo Alto, CA). The masses of the eluted fractions were analyzed on an ESI-MS single quadrupol spectrometer PE SCIEX API 165 (Applied Biosystems Perkin-Elmer Corp., Foster City, CA).

**1.4. Expression and purification of the Met, Aha and Hpg variants of hEGF.** Plasmids pQE80L-R2ψhEGF, pQE80L-G2ψhEGF and pET15b-hEGF for the expression of ψhEGF mutants R2 M22I; R2G M22I; and τhEGF, respectively, were transformed into the Met-auxotrophic *Escherichia coli* strain B834(DE3) (F*⁻ompT hsdS_{D}*(*r_{D}⁻ m_{D}⁻*) *gal dcm metE* (DE3)) (Novagen Merck Chemicals Ltd., Nottingham, UK) following standard protocols. For the expression of Met→Hpg and Met→Aha substituted hEGF mutants as well as the natural Met variants in inclusion bodies, we followed previously described protocols ⁽²⁴⁾. Briefly, cells were grown to mid-log phase (OD₆₀₀ 0.6-0.8) in synthetic medium containing limiting amounts (5 mg/l) of Met. Met-starved cells were induced for protein expression (IPTG) with concomitant addition of either 50 mg/l L-Met or 100 mg/l of L-Hpg or L-Aha. The inclusion body isolation and refolding protocol was identical for all mutant hEGF variants. Cells were first harvested and lysed in lysis buffer (50 mM NaH₂PO₄, 300 mM NaCl, pH 8.0). After addition of lysozyme, DNAse and RNAse, the suspension was sonicated and subsequently spun down at 63000 x g, 4 °C for 45 min. The pellet with inclusion bodies was washed twice with lysis buffer and resuspend in urea buffer (8 M urea, 5 mM DTT, 50 mM Tris-HCl pH 8.0). Insoluble material was removed by centrifugation at 63000 x g, 4 °C for 30 min. The supernatant was dialyzed first against 400 mM arginine, 50 mM Tris-HCl pH 8.0 for at least 4 h and then against two changes of 50 mM Tris-HCl pH 8.0. All dialysis steps were performed at room temperature. After another spin as described above, the supernatant containing the desired renatured ψhEGF variant and residual cellular proteins was loaded onto HiTrap Q-sepharose (GE Healthcare Bio-Sciences AB, Uppsala, Sweden), washed with 50 mM Tris-HCl pH 8.0 and eluted with a gradient of 0-1 M NaCl in 50 mM Tris-HCl pH 8.0. ψhEGF variants were purified on Ni-NTA resin (Qiagen, Hilden, Germany) following standard protocols. Elution fractions were analyzed by SDS-PAGE and those enriched in the desired hEGF variant were pooled and lyophilized.

**1.5. Analysis of hEGF variants.** The lyophilized protein samples were redissolved in water to a final concentration of approximately 1-2 mg/ml. HPLC was performed on a YMC-Pack Protein-RP, 250 x 4.6 mm , S-5 µm column (YMC Europe GmbH, Dinslaken, Germany) using the same HPLC system as described above for peptide analysis. However, fractions were collected manually. High resolution mass spectrometry of selected HPLC fractions was performed on a Micro-TOF-LC mass spectrometer (Bruker Daltonics, Bremen, Germany). HPLC purified protein was injected directly in a carrier flow of 30 % A (0.05 % TFA in water) and 70 % B (0.05 % TFA in acetonitrile) at a rate of 250 µl/min.

### 2. Results

We studied the effects of azidohomoalanine (Aha) and Hpg (Fig. 1B) on N-terminal amino acid excision, which have chemically unique azido and alkyne side chains.

In order to test catalytic efficiency of MetAP towards these analogs we prepared a series of pentapeptides containing Met, Hpg or Aha as N-terminal amino acids with Arg or Gly in the second position (penultimate residues).

The overnight incubation of Arg2 penatpeptides (Met1-Arg2-Gln3-Leu4-Phe5; Aha1-Arg2-Gln3-Leu4-Phe5; and Hpg1-Arg2-Gln3-Leu4-Phe5) with recombinant *E. coli* MetAP yielded no cleavage of Met, nor Aha or Hpg from the peptides (data not shown). That means, the excision was blocked completely by Arg in the second position. On the other hand, the excision of Met, Aha and Hpg was observed in the case of Gly2 pentapeptides (Met1-Gly2-Gln3-Leu4-Phe5; Aha1-Gly2-Gln3-Leu4-Phe5; and Hpg1-Gly2-Gln3-L4-Phe5). The canonical amino acid Met was fully cleaved after 10 minutes whereas complete excision of Aha and Hpg was observed only after 100 and 250 minutes, respectively (Figure 2). Although Aha and Hpg are less efficiently processed than Met in Gly2 pentapeptides, they are indeed substrates for *E. coli* MetAP. Nonetheless, the main differences are found in the excision kinetics. These allowed us to identify the following order of the *in vitro* cleavage efficiency in Gly2 pentapeptides: Met > Aha > Hpg.

Interestingly, the Aha-pentapeptide was better processed by the *E.coli* MetAP than the Hpg-pentapeptide, while we observed the opposite with MetAP from *Pyrococcus furiosus* (data not shown). Obviously, the substrate specificities of prokaryotic and archaeal MetAPs are different.

To identify possible differences in the efficiency of N-terminal residue cleavage during recombinant protein expression in *E. coli*., we chose the human epidermal growth factor (hEGF, Figure 1A) as a model protein. Mature hEGF is generated by proteolytic cleavage of a precursor protein and plays a crucial role in the regulation of cell growth, proliferation and differentiation. Biobioorthogonal transformation of the hEGF N-terminus by, e.g., fluorescent labeling is highly desirable to facilitate examination of its biological activities at the molecular level. Therefore, it is of outstanding interest to assess the N-terminal configuration upon incorporation of Met analogs with biobioorthogonal groups, such as Aha or Hpg. We mutated the single Met22 of mature hEGF into Ile which yielded in a recombinant protein composed of 55 amino acids termed pseudo-hEGF (ψhEGF). Its single N-terminal Met is contiguous to Arg and Asn as the penultimate and antepenultimate residues, respectively (Met1-Arg2-Asn3-ψhEGF) (Figure 3).

For successful Met → Met analog replacement in Met1-Arg2-Asn3-ψhEGF, Met-auxotrophic *E.coli* B834(DE3) host cells harboring the expression construct under the control of the T5 promotor were grown in synthetic minimal medium supplemented with 5 mg/l L-Met as the natural substrate. After exhaustion of Met, 100 mg/l Hpg or Aha were added and the expression was induced with IPTG. Met1-Arg2-Asn3-ψhEGF and the variants Aha1-Arg2-Asn3-ψhEGF and Hpg1-Arg2-Asn3-ψhEGF (Figure 3) were equally well expressed. Although all proteins were expressed in inclusion bodies, they were successfully solubilized by dialysis against arginine. All variants were enriched by ion exchange chromatography on Q-sepharose and purified by subsequent reversed phase HPLC (see Experimental Section for details). Since the molecular mass difference between Met and Aha or Hpg is sufficiently large (-5 Da and -22 Da, respectively) to be determined experimentally, we performed mass spectrometric analysis of the purified Met, Aha and Hpg variants (Table 1). We exclusively found the masses of the intact proteins, i.e., none of the N-terminal residues had been excised due to the bulkiness of penultimate Arg residue. In addition, we never observed mass peaks corresponding to formylated Met, Aha or Hpg.

In order to facilitate excision of the N-terminal residue the penultimate Arg residue in Met1-Arg2-Asn3-ψhEGF was mutated to Gly, yielding Metl-Gly2-Asn3-ψhEGF (Figure 3). The Met, Aha and Hpg variants were expressed and purified as described above. To our surprise, both Metl-Gly2-Asn3-ψhEGF and Hpg1-Gly2-Asn3-ψhEGF were poorly expressed, whereas the expression of Aha1-Gly2-Asn3-ψhEGF was slightly better. In spite of the dramatically impaired expression we were able to isolate low amounts of all three variants for mass analysis (Table 1). N-terminal Aha was inefficiently cleaved (∼30 %); whereas the Hpg variant had an intact N-terminus. Most contradictorily, Met was cleaved only to ∼3 %. Obviously, Gly as the penultimate residue in the ψhEGF variants is extremely unfavorable not only for protein expression but also for N-terminal residue excision. While Met and Aha experienced some cleavage; N-terminal Hpg remained untouched.

The fact that Aha was better processed than Hpg is in agreement with our observations that Aha is generally better biocompatible than Hpg and other Met analogs (with the exception of selenomethionine).

The negligible excision of Met from Met1-Gly2-Asn3-ψhEGF under the described expression conditions could be explained by earlier observations that in some proteins, Gly in the penultimate position does not support N-terminal Met excision (16). This might be due to the fact that Gly has no optically active α-carbon and can exist in conformations inaccessible to other amino acids. On the other hand it might well be that the N-termini of the variants are inaccessible during ribosomal translation. Most probably, they are trapped in aggregated or inclusion body form leaving no time for N-terminal residue excision. This assumption is supported by our observation that purified, refolded Met1-Gly2-Asn3-ψhEGF was readily cleaved by *E.coli* MetAP.

NME is a co-translational process and MetAP starts processing as soon as the N-terminus of the nascent polypeptide protrudes form the ribosome tunnel ⁽³⁾. In eukaryotic cells, this mode of action coincides with the intracellular localization of MetAP close to the ribosome (17). The localization of bacterial MetAP is still unclear. Since, native, mature hEGF contains a β-sheet structural element quite close to the N-terminus (Figure 1), we hypothesized that an unstructured tag at the N-terminus of hEGF would provide a better system to observe co-translational MetAP-mediated cleavage, even though the protein might still be trapped in insoluble form.

In our model protein re-design we left out the Met22Ile mutation of ψhEGF and cloned the coding sequence for the mature wild-type hEGF into the T7-based pET15b expression vector so that the protein would be expressed with additional 21 amino acids at its N-terminus (Figure 3). This tag contains a hexahistidine stretch followed by a thrombin cleavage site and is commonly used for high yield expression and purification of recombinant proteins. There are three Met present in the tagged hEGF mutant, Met1 and Met21 within the N-terminal tag and Met43, which corresponds to Met22 in mature hEGF. The N-terminus is composed of Met in the first position and Gly and Ser as the penultimate and antepenultimate residues, respectively. The mutant protein was termed Met1-Gly2-Ser3-ThEGF (Figure 3), where τ signifies the N-terminal 21 amino acid tag. We expected that Gly and Ser in the vicinity of the N-terminal Met or Aha and Hpg residues would facilitate NME.

Met1-Gly2-Ser3-ThEGF, Aha1-Gly2-Ser3-ThEGF and Hpgl-Gly2-Ser3-ThEGF were expressed in inclusion bodies and purified as outlined above except that Ni-chelate chromatography was applied instead of ion exchange chromatography. Mass spectrometry (Figure 4) of the Met variant revealed that only about 10% of the proteins contained an initial Met residue (expected mass: 8667; found mass: 8667.5 Da), whereas the N-terminally processed species dominated (expected mass: 8536; found mass: 8536.7 Da; Figure 4A). In contrast, the Hpg variant yielded ∼2/3 of the protein containing 3 Hpg (expected mass 8601 Da; found mass: 8601.8 Da) and ∼1/3 of the protein with only two Hpg (expected and found mass: 8492.0 Da) or other combinations of Hpg and Met (Figure 4B). This strongly indicates an efficient blockage of N-terminal Hpg excision.

| **Table 1.** Mass spectrometric analysis of the different Met, Aha and Hpg variants after *in vivo* NME. | | |
|---|---|---|
| Variant | calculated mass | found mass |
| | / Da | / Da |
| Met1-Arg2-Asn3- | 6485 | 6484.9000 |
| ψhEGF | | |
| Arg2-Asn3-ψhEGF | 6354 | nd |
| Aha1-Arg2-Asn3- | 6480 | 6479.8996 |
| ψhEGF | | |
| Arg2-Asn3-ψhEGF | 6354 | ta |
| Hpg1-Arg2-Asn3- | 6463 | 6462.8994 |
| ψhEGF | | |
| Arg2-Asn3-ψhEGF | 6354 | nd |
| Met1-Gly2-Asn3- | 6386 | 6385.7976 |
| ψhEGF | | |
| GIy2-Asn3-ψhEGF | 6255 | 6254.6831^{[a]} |
| Aha1-Gly2-Asn3- | 6381 | 6384.8054 |
| ψhEGF | | |
| Gly2-Asn3-ψhEGF | 6255 | 6254.8090^{[b]} |
| Hpg1-Gly2-Asn3- | 6364 | 6363.7944 |
| ψhEGF | | |
| Gly2-Asn3ψ-hEGF | 6255 | nd |
| [a] excision -3 % [b] excision -30 %; % excision relate to the corresponding variant with the intact N-terminus. Nd, not detectable; ta, trace amounts. Corresponding mass spectra are shown in the Supporting Information. | | |

Unfortunately, due to difficulties with Ni-chelate chromatography, we could analyze masses of only minute amounts of semi-pure Aha1-Gly2-Ser3-τ hEGF. Besides noticeable contamination with unprocessed (3 Met; expected mass: 8667 ; found mass: 8665.8 Da) and processed (2 Met; expected mass: 8536 ; found mass: 8534.8 Da) Met1-Gly2-Ser3-τhEGF, we observed equal amounts of the variant containing either three (expected mass: 8652 Da; found mass: 8649.7 Da) or two Aha (expected mass: 8526 Da; found mass: 8524.9 Da) (Figure 4C). The reproducible 1:1 ratio of processed to unprocessed Aha-proteins correlates well with our observation that Aha1-Gly2-pentapeptides were more efficiently processed than Hpgl-Gly2-pentapeptides (Figure 2). In addition, protein species with 2,4-diaminobutyric acid (DAB) as a result of Aha reduction were detected (Figure 4C).

The experiments presented in this study provide first evidence that the NME rules can be extended by an appropriate combination of N-terminal sequence composition with the chemical nature of the initiator residue. While Arg also efficiently inhibited *in vivo* excision of N-terminal Met, Aha and Hpg in our model protein, ψhEGF, the results with Gly as the penultimate residue were more complex. Besides noticeably low expression, in none of the Met, Aha or Hpg variants the N-terminal residue was efficiently cleaved. Only after elongation by 21 amino acids and alteration of the N-terminal sequence composition of the protein (Met/Aha/Hpgl-Gly2-Ser3), we observed NME whose efficiency resembled that with the Gly2-pentapeptides, i.e. Met > Aha > Hpg.

The general applicability of our observations should be tested in future experiments including other proteins, N-terminal sequence compositions and other non-canonical amino acids. For example, by using soluble model proteins the experimental setup certainly affects NME, e.g., during the preparation of cell lysates MetAP might be delocalized and act on all the (soluble) proteins in the lysate. Met analogs that more or less efficiently block NME, such as trifluoro-Met, Hpg or Aha, appear to be the paradigm of a new class of MetAP inhibitors that are an integral part of the protein N-terminus. In mammalian cells, NME is indispensable for the posttranslational modification of N-terminal amino acids, such as N-myristoylation, N-acetylation, N-methylation or O-phosphorylation ^{(18,19)}. Therefore, the possibility to specifically insert Aha or Hpg at the N-terminus will provide a chemical handle for subsequent novel biobioorthogonal chemical transformations. Previously, Varshavsky elaborated the mechanism that links protein half-life to the nature of its N-terminal amino acid ('N-end rule')^{(20,21)}. We anticipate that by chemical post-translational modifications one could afford an efficient control of the N-end rule pathways, i.e. the stability, life-span and metabolic fate of a target protein The removal of N-terminal Met is dictated by the nature and bulkiness of the side chain of the second amino acid. NME requires at least Met aminopeptidase (MAP; EC 3.4.11.18) activity.

The associated crucial physiological consequences, such as protein stabilization associated with Met removal, highlight the importance of correctly predicting the N termini of proteins as recently reported for higher eukaryotes. Mastering NME in these cells will enable us to control protein production in recombinant host cells such as eubacteria, archea or eukaryotes, making it possible to produce drugs with the most active N-terminal configurations possible.

Bacterial MAPs are targets for antimicrobial therapy, (37) whereas the structurally similar human MAPs are targets for anticancer agents (38); therefore, discovery of new inhibitors offers hope for treatment of microbial infections as well as cancer.

### List of References

(1) C. Giglione, A. Boularot, T. Meinnel, Cell. Mol. Life Sci. 2004, 61, 1455
(2) A. Benbassat, K. Bauer, S.Y. Chang, K. Myambo, A. Boosmann,S. Chang, J. Bacteriol., 1987, 169, 751
(3) R.A. Bradshaw, W.W. Brickey, K.W. Walker, Trends Biochem. Sci., 1998, 23, 263
(4) C. Flinta, B. Persson, H. Jornvall, G. Vonheijne, Eur. J. Biochem, 1986, 154, 193
(5) F. Frottin, A. Martinez, P. Peynot, S. Mitra, R.C. Holz, C. Giglione, T. Meinnel, Mol. Cell. Proteomics, 2006, 5, 2336
(6) J. Solbiati, A. Chapman-Smith, J.L. Miller, C.G. Miller, J.E. Cronan, J. Mol. Biol., 1999, 290,607
(7) N. Budisa, C. Minks, S. Alefelder, W. Wenger, F.M. Dong, L. Moroder, R. Huber, FASEB J. 1999, 13, 41
(8) K.L. Kiick, R. Weberskirch, D.A. Tirrell, FEBS Lett. 2001, 502, 25
(9) N. Budisa, Angew. Chem. Int. Ed. Engl. 2004, 43, 6426
(10) A.J. Link, D.A. Tirrell, Methods 2005, 36, 291
(11) K.L. Kiick, J.C.M. Van Hest, D.A. Tirrell, Angew. Chem. Int. Ed. Engl. 2000, 39, 2148
(12) J.C.M. Van Hest, K.L. Kiick, D.A. Tirrell, J.Am.Chem.Soc. 2000, 122, 1282
(13) N. Budisa, O. Piptone, I. Siwanowicz, M. Rubini, P.P.Pal, T.A. Holak, M.L. Gelmi, Chem. Biodivers. 2004, 1, 1465
(14) N. Budisa in Engineering the Genetic Code, Wiley, Weinheim, 2005, pp.90
(15) G. Carpenter, S. Cohen, J.Biol.Cehm. 1990, 265, 7709
(16) A. Ben-Bassat, K. Bauer, S.Y. Chang, K. Myambo, A. Boosmann, S. Chang, J. Bacteriol. 1987,169,751-757
(17) J.A. Vetro, Y.H. Chang, J. Cell. Biochem. 2002,85,678
(18) R.J. Duronio, D.A. Towler, R.O. Heuckerodth, J.I. Gordon, Science, 1989, 243, 796
(19) C. Giglione, O. Vallon, T. Meinnel, EMBO J., 2003, 22,13
(20) A. Varshavsky, Cell 1992,69,725
(21) A. Varshavsky, Proc. Natl. Acad. Sci. U.S.A.,1996,93,12142
(22) J.B. Mangold, M.R. Mischke, J.M. LAVelle, Mutat.Res. 1989, 216,27
(23) Y.-D. Liao, J.-C.Jeng, C.-F.Wang, S.-C.Wang, S.-T.Chang, Protein Sci. 2004, 13, 1802
(24) N. Budisa, B. Steipe, P.Demange, C. Eckerskorn, J. Kellermann, R. Huber, Eur.J.Biochem.,1995,230,788
(25) Taki M., Sawata S.Y., Taira K (2001). Specific N-terminal biotinylation of a protein in vitro by a chemically modified tRNAfmet can support the native activity of the translated protein. J. Biosci. Bioeng. 92, 149-153.
(26) Olejnik, J., Gite, S., Mamaev, S. and K. J. Rothschild (2005). N-terminal labeling of proteins using initiator tRNA. Methods, 36, 252-260.
(27) Taki, M., Kuno, A., Matoba, S., Kobayashi, Y., Futami, J., Murakami, H., Suga, H., Taira, K., Hasegawa, T. and M. Sisido (2006). Leucyl/Phenylalanyl-tRNA-protein transferase-mediated chemoenzymatic coupling of N-terminal Arg/Lys units in posttranslationally processed proteins with non-natural amino acids. ChemBioChem, 7, 1676-1679.
(28) Schimizu, Kanamori, Ueda (2005). Protein Synthesis by Pure Translation System. Methods 36: 299-304.
(29) Amstutz, Forrer, Zahnd, Plünckthun (2001). In Vitro Display Technologies. Current Oppinion in Biotechnology 36: 400-405.
(30) He, Taussig (2002). Ribosome Display: Cell free protein display technology. Briefings in Functional Genomics and Proteomics 1: 204-212.
(31) Tan, Blacklow, Cornisch, Forster (2005). De Novo Genetic Codes and Pure Translation Display. Methods 36: 277-290.
(32) Josephson, Hartmann, Szostak (2005). Ribosomal Syntheis of Unnatural Peptides. J. Am. Chem. Soc. 127: 11727-11735.
(33) Hunt, S. (1991). Non-Protein Amino Acids. In Amino acids, proteins and nucleic acids (Dey, P., Harborne, J. & Rogers, L., eds.), Vol. 5. Academic Press.
(34) Rosenthal, G. (1982). Plant nonprotein amino and imino acids. Biological, Biochemical and Toxicological Properties, Academic Press, New York.
(35) Varshavsky, A. (1996). The N-end rule: Functions, mysteries, uses. Proc. Natl. Acad. Sci. USA, 93, 12142-12148.
(36) A. Varshavsky, A. (1992). The N-End Rule. Cell, 69, 725-735.
(37) Vaughan MD, Sampson PB, Honek JF. Methionine in and out of proteins: targets for drug design. Curr Med Chem 2002; 9: 385-409
(38) Ingber D, Fujita T, Kishimoto S, Sudo K, Kanamaru T, Brem H, Folkman J. Synthetic analogues of fumagillin that inhibit angiogenesis and suppress tumour growth. Nature 1990; 348: 555-557; Logothetis CJ, Wu KK, Finn LD, Daliani D, Figg W, Ghaddar H, Gutterman JU. Phase I trial of the angiogenesis inhibitor TNP-470 for progressive androgen-independent prostate cancer. Clin Cancer Res 2001; 7: 1198-1203.

## Claims

1. A method of producing an N-terminally modified polypeptide by translation from an RNA molecule in a host cell or in an in vitro translation system, wherein the RNA molecule encodes a polypeptide comprising an N-terminal methionine residue, wherein the translation occurs at conditions under which the N-terminal methionine residue is replaced by a non-genetically encoded amino acid residue and wherein thereby an N-terminal amino acid excision of the polypeptide is modified.

2. The method of claim 1, wherein the host cell is a bacterial cell, particularly a gram-negative bacterial cell such as E. coli.

3. The method of claim 1 or 2, wherein the host cell is a methionine-auxotrophic host cell.

4. The method of any one of claims 1-3, wherein the polypeptide comprises a single methionine residue, which is the N-terminal methionine residue.

5. The method of claims 1-3, wherein the polypeptide comprises an N-terminal methionine residue and at least one internal methionine residue.

6. The method of claim 5, wherein only the N-terminal methionine residue is replaced by a non-genetically encoded amino acid.

7. The method of claim 5, wherein the N-terminal methionine residue and at least one internal methionine residue are replaced by non-genetically encoded amino acid residues.

8. The method of any one of claims 1-7, wherein the N-terminal amino acid excision is increased.

9. The method of any one of claims 1-7, wherein the N-terminal amino acid excision is decreased.

10. The method of any one of claims 1-9, wherein the penultimate amino acid residue after the N-terminal amino acid residue is an amino acid residue which affects N-terminal amino acid excision and/or polypeptide stability.

11. The method of claim 10, wherein the penultimate amino acid residue is an amino acid residue which promotes N-terminal amino acid excision, particularly selected from the group consisting of Gly, Ala, Pro, Cys, Ser, Thr and Val.

12. The method of claim 10, wherein the penultimate amino acid residue is an amino acid residue which increases polypeptide stability, particularly selected from the group consisting of Met, Gly, Ala, Ser, Thr and Val.

13. The method of claim 10, wherein the penultimate amino acid residue is an amino acid residue which inhibits N-terminal amino acid excision, particularly selected from the group consisting of Arg, Lys, Phe, Leu and Ile.

14. The method of claim 10, wherein the penultimate amino acid residue is an amino acid residue which decreases polypeptide stability, particularly selected from the group consisting of Arg, Lys, Asp, Leu, Ile and Phe.

15. The method of any one of claims 1-14, wherein the ante-penultimate amino acid residue after the N-terminal amino acid residue is an amino acid residue which affects N-terminal amino acid excision and/or polypeptide stability.

16. The method of claim 15, wherein the ante-penultimate amino acid residue is selected from Glu, Asp, Thr, Leu, Ile or Val.

17. The method of any one of claims 1-16, wherein the non-genetically encoded amino acid residue comprises a functional chemical moiety.

18. The method of claim 17, wherein the functional chemical moiety is selected from the group consisting of azide, alkene, alkyne, phosphine, halogen, or chalcogen, amine or ester moieties.

19. The method of claim 18, wherein the functional chemical moiety is an aromatic and/or aliphatic azide or alkyne group.

20. The method of any one of claims 1-19, wherein the non-genetically encoded amino acid residue is homopropargylglycine (Hpg) or azido homoalanine (Aha).

21. The method of any one of claims 1-20, further comprising coupling a heterologous molecule to the functional chemical moiety of the non-genetically encoded amino acid residue.

22. The method of claim 11, wherein the coupling comprises a [1,3]-dipolar cyclo-addition between an azide group and an alkyne group.

23. The method of any one of claims 12 or 13, wherein the heterologous molecules are selected from the group consisting of reporter molecules, effector molecules, solid phases and nanomaterials.

24. The method of any one of claims 1-23, wherein the polypeptide is selected from growth factors, viral antigens, annexins, antibodies, structure proteins, barstar, G-protein coupled receptors, green fluorescent protein and its spectral variants, human superoxide dismutase (hSOD1), spider silk and extracellular matrix proteins, proteases, kinases, phosphatases, hydroxylases, ligases or polymerases.

25. A method of producing an N-terminally modified polypeptide by translation from an RNA molecule in a host cell or in an in vitro translation system, wherein the RNA molecule encodes an N-terminal polypeptide portion comprising an amino acid sequence which allows proteolytic cleavage and wherein the first amino acid residue behind the cleavage site is the amino acid residue to be modified, wherein the translation occurs at the conditions under which the amino acid residue to be modified is replaced by a non-genetically encoded amino acid residue, and wherein the polypeptide is subjected to a proteolytic cleavage at the cleavage site.

26. The method of claim 25 wherein the amino acid residue to be modified is a methionine residue.

27. The method of claim 25 or 26, wherein the amino acid residue to be modified only occurs once in the polypeptide after cleavage of the N-terminal polypeptide portion.

28. A method of modifying efficacy of translation initiation, wherein an RNA molecule encoding a polypeptide comprising an N-terminal amino acid residue is translated in a host cell or in an in vitro translation system, wherein the translation occurs at conditions under which the N-terminal methionine residue is replaced by a non-genetically encoded amino acid residue and wherein thereby the efficacy of translation initiation is modified, e.g. decreased or increased.

29. An N-terminally modified polypeptide comprising at its N-terminus a non-genetically encoded amino acid residue, obtainable by the method of any one of claims 1-28.

30. The polypeptide of claim 29, wherein the non-genetically encoded amino acid residue comprises a functional chemical moiety.

31. The polypeptide of claim 30, wherein the functional chemical moiety is coupled to a heterologous molecule.
